# EUROPEAN PATENT APPLICATION

(11) **EP 2 322 241 A2**
(43) Date of publication of application: **18.05.2011**
(21) Application number: 10169291.1
(22) Date of filing: 12.07.2010
(51) Int. Cl.: A61M 16/08, A61M 11/06

(54) **Tee connector for supplying aerosol**

(30) Priority: 16.11.2009 TW 098138807
(71) Applicant: Galemed Corporation, I-Lan (TW)
(72) Inventor: Ma, Jun, Fujian Province (CN); Lee, Gary C. J., 268, I-Lan (TW); Lee, Ching-Hui, 265, I-Lan (TW)
(74) Representative: Stöckeler, Ferdinand

(57) **Abstract**

A Tee connector (100) for supplying aerosol includes: a Tee-shaped tube (1) having a transverse tube portion (11) and a longitudinal tube portion (12), the transverse tube portion (11) having inlet and outlet ports and being formed with a top opening (111) and a bottom opening (121), the bottom opening (121) being aligned with the top opening (111) and being connected fluidly to the longitudinal tube portion (12); a cover (2) closing detachably the top opening (111); a valve (4) including a valve plate (41) which is movable downward to close the bottom opening (121) and upward to open the bottom opening (121); and a spring (3) disposed within the transverse tube portion (11) and urging the valve plate (41) so that the valve plate (41) is moved downward to close the bottom opening (121).

## Description

This invention relates to a Tee connector, more particularly to a Tee connector for supplying aerosol.

A conventional Tee connector 9 for supplying aerosol, as shown in Fig. 1, is disclosed in US 6,725,858 B2, and includes a Tee-shaped tube 91 and a valve device 92 disposed in the Tee-shaped tube 91. The Tee-shaped tube 91 has a transverse tube portion 911 and a longitudinal tube portion 912 that is connected fluidly to the transverse tube portion 911 through a connecting hole. The valve device 92 has a valve member 921, a sliding member 922 disposed below and connected to the valve member 921, and a spring 923. The sliding member 922 is disposed in the longitudinal tube portion 912. A lower portion of the valve member 921 is connected to the sliding member 922, and an upper portion of the valve member 921 can close the connecting hole. The spring 923 is disposed in the longitudinal tube portion 912 and urges the sliding member 922 downward such that the connecting hole is normally closed by the upper portion of the valve member 921, and such that the transverse tube portion 911 and the longitudinal tube portion 912 are normally disconnected fluidly.

When the Tee connector 9 is used for supplying aerosol, a nebulizer (not shown) is inserted from a bottom end of the longitudinal tube portion 912 to push the sliding member 922 and the valve member 921 to move upward so as to open the connecting hole. The transverse tube portion 911 fluidly connects a gas supplier to a mouth of a patient. The nebulizer supplies the aerosol containing medical preparation to the transverse tube portion 911 via the longitudinal tube portion 912.

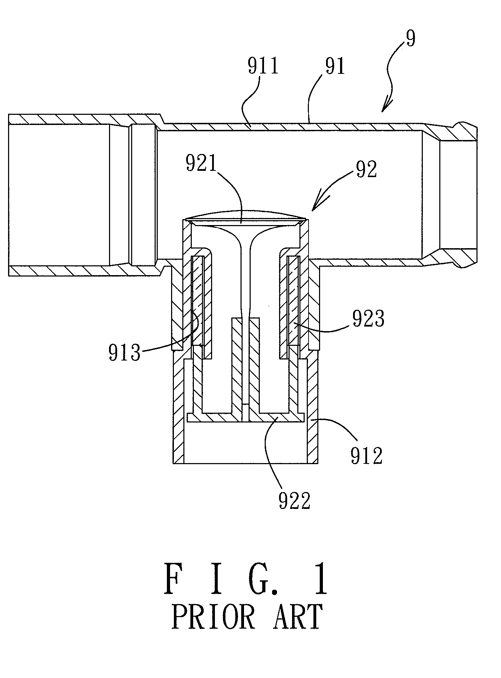

However, when the valve device 92 is assembled into the Tee-shaped tube 91, as shown in Fig. 1, the spring 923 should be firstly fitted into a gap 913 in the longitudinal tube portion 912, and then, the valve member 921 and the sliding member 922 are assembled into the longitudinal tube portion 912 from two ends of the longitudinal tube portion 912. Finally, the transverse tube portion 911 and the longitudinal tube portion 912 are assembled together via an ultrasonic bonding process. Therefore, assembly of the Tee connector 9 is relatively complicated.

Therefore, an object of the present invention is to provide a Tee connector for supplying aerosol, which can be easily assembled.

Accordingly, a Tee connector for supplying aerosol of the present invention comprises:
a Tee-shaped tube having a transverse tube portion and a longitudinal tube portion, the transverse tube portion having inlet and outlet ports and being formed with a top opening and a bottom opening, the bottom opening being aligned with the top opening and being connected fluidly to the longitudinal tube portion;
a cover closing detachably the top opening;
a valve including a valve plate which is movable downward to close the bottom opening and upward to open the bottom opening; and
a spring disposed within the transverse tube portion and urging the valve plate so that the valve plate is moved downward to close the bottom opening.

Other features and advantages of the present invention will become apparent in the following detailed description of the preferred embodiment of the invention, with reference to the accompanying drawings, in which:
Fig. 1 is a cross-sectional view of a conventional Tee connector;
Fig. 2 is an exploded view of a Tee connector according to the present invention;
Fig. 3 is a sectional plan view of the Tee connector according to the present invention;
Fig. 4 is a sectional elevation view of the Tee connector according to the present invention; and
Fig. 5 is a sectional elevation view showing that the Tee connector according to the present invention is connected to a nebulizer.

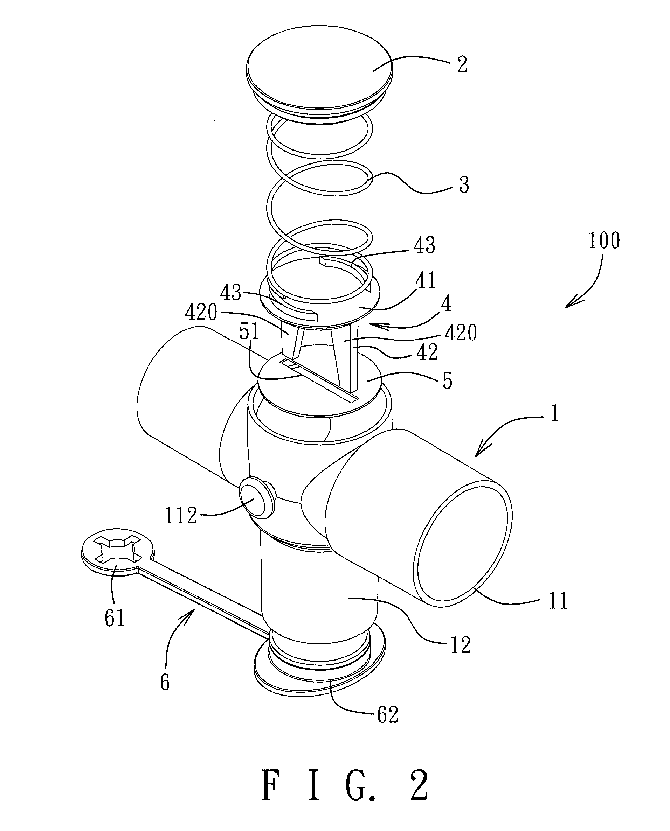

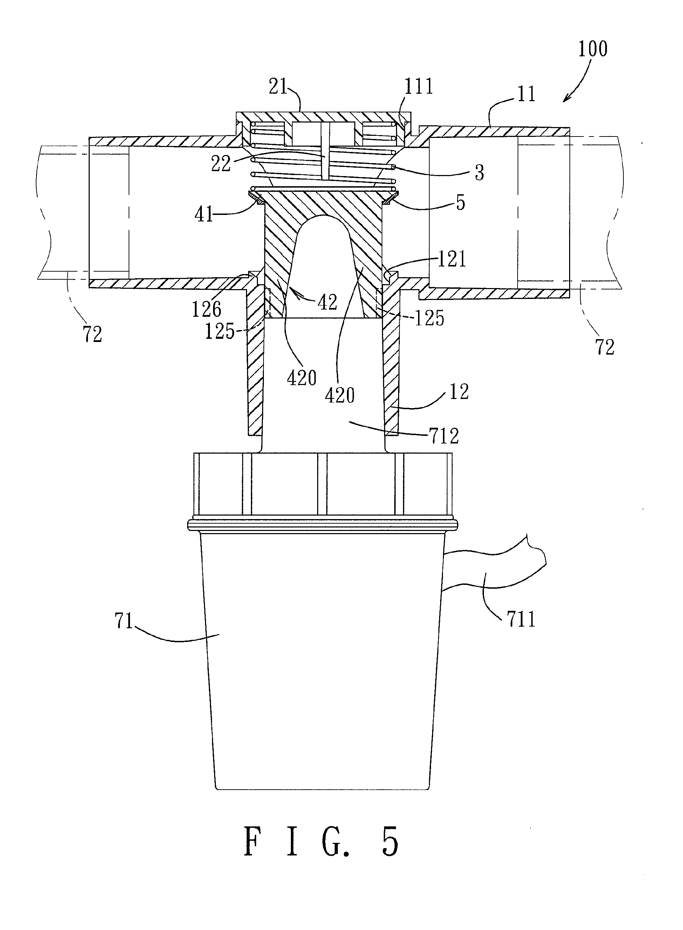

Referring to Figs. 2 and 5, a Tee connector 100 according to the preferred embodiment of this invention can be connected to a nebulizer 71 for supplying aerosol and can also be connected to two conduits 72 for supplying gas (for example, oxygen) to a patient. The aerosol of the nebulizer 71 contains medical preparation and is supplied to the patient through the Tee connector 100 and the conduits 72. The Tee connector 100 comprises a Tee-shaped tube 1, a cover 2, a spring 3, a valve 4, and a gasket 5.

As shown in Figs. 2 to 5, the Tee-shaped tube 1 has a transverse tube portion 11 and a longitudinal tube portion 12. The transverse tube portion 11 has inlet and outlet ports that can be respectively connected to the conduits 72. The transverse tube portion 11 is formed with a top opening 111 and a bottom opening 121 between the inlet and outlet ports. The bottom opening 121 is aligned with the top opening 111 and is connected fluidly to the longitudinal tube portion 12.

In the preferred embodiment, the transverse tube portion 11 has a valve seat 126 disposed around the bottom opening 121 and protruding from an inner wall of the transverse tube portion 11.

As shown in Fig. 2, the spring 3, the valve 4 and the gasket 5 are all assembled inside the Tee-shaped tube 1 through the top opening 111.

The valve 4 includes a valve plate 41, a valve extension 42 and two angularly extending arc-shaped flanges 43.

The valve plate 41 is movable downward to close the bottom opening 121 and upward to open the bottom opening 121. The valve extension 42 extends from the valve plate 41 into the longitudinal tube portion 12. The angularly extending arc-shaped flanges 43 protrude upwardly from a top face of the valve plate 41 at diametrically opposite positions.

The valve plate 41 is normally seated on the valve seat 126 and is moved downward to close the bottom opening 121. That is to say, the transverse tube portion 11 and the longitudinal tube portion 12 are not connected fluidly unless the valve plate 41 is moved upward and away from the valve seat 126.

Preferably, the valve extension 42 includes two extension legs 420 that are guided slidably by the longitudinal tube portion 12.

The spring 3 is a coiled spring or a compression spring and is disposed within the transverse tube portion 11 to urge the valve plate 41. Therefore, the valve plate 41 normally closes the bottom opening 121. In assembly, the spring 3 is installed through the top opening 111 such that a bottom end of the spring 3 is disposed around and contacts the arc-shaped flanges 43. Then, the top opening 111 is closed detachably by the cover 2 such that a top end of the spring 3 is pressed by a top plate 21 of the cover 2. In other words, the top and bottom ends of the spring 3 respectively abut against the top plate 21 and the valve plate 41.

Preferably, the spring 3 has a diameter larger than an inner diameter of the transverse tube portion 11. Accordingly, the spring 3 will not easily change in position within the transverse tube portion 11 to obstruct a gas flow in the transverse tube portion 11.

The cover 2 includes the top plate 21, a pin portion 22, and a surrounding wall 23. The top plate 21 is for closing the top opening 111. The pin portion 22 downwardly extends from the top plate 21 toward the valve 4. The surrounding wall 23 extends downwardly from the top plate 21 around the pin portion 22 and is fitted into the top opening 111.

Preferably, an outer surface of the surrounding wall 23 may be provided with a hook-like structure for hooking with the transverse tube portion 11 around the top opening 111, or an interference structure to create an interference engagement when the top plate 21 closes the top opening 111. The pin portion 22 has a bottom end extending beyond the surrounding wall 23 to contact and limit an upward movement of the valve plate 41 when the valve plate 41 moves upward. Therefore, when the valve plate 41 is pushed upward, it can reach only the bottom end of the pin portion 22. As such, the valve plate 41 will not move to the surrounding wall 23, thereby preventing the valve plate 41 from being stuck in the surrounding wall 23.

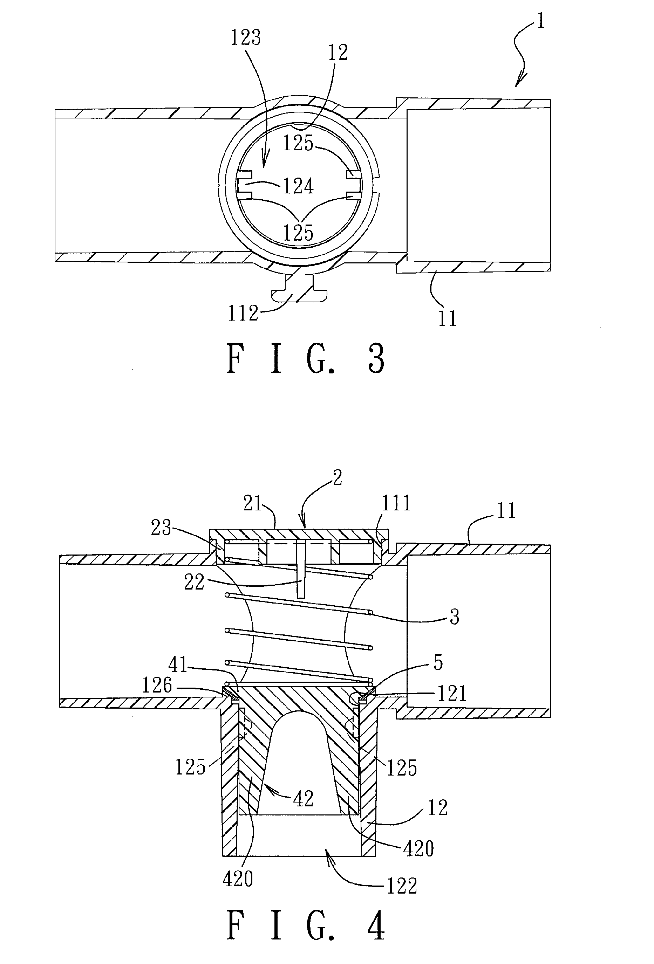

Referring back to Figs. 4 and 5, in use, the two conduits 72 are respectively connected to two ends of the transverse tube portion 11. One of the conduits 72 is connected to a gas supplying apparatus (not shown) and another one is connected to the mouth of the patient. The nebulizer 71 is connected to a bottom end 122 of the longitudinal tube portion 12.

In this embodiment, the nebulizer 71 has an insert tube 712 that is a narrow tube extending upwardly and that can be connected to the Tee-shaped tube 1 by inserting the insert tube 712 into the longitudinal tube portion 12. When the insert tube 712 is inserted into the longitudinal tube portion 12, it will contact and push the valve extension 42 to slide upward and to compress the spring 3. At the same time, the valve plate 41 will move upward to open the bottom opening 121. Accordingly, the longitudinal tube portion 12 can be fluidly connected to the transverse tube portion 11. Besides, the nebulizer 71 is usually connected to a gas conduit 711, and thus, the aerosol of the nebulizer 71 can flow together with the gas from the gas conduit 711 into the mouth of the patient through the Tee-shaped tube 1.

For ensuring that the valve 4 can move smoothly relative to the longitudinal tube portion 12, the longitudinal tube portion 12 has a guiding member 123 for guiding slidably the valve 4(see Fig. 3). For cooperating with the structure of the valve extension 42 of the valve 4, the guiding member 123 has two guiding grooves 124 formed on an inner wall of the longitudinal tube portion 12 to guide the extension legs 420, respectively. Each of the guiding grooves 124 is confined by a pair of parallel flanges 125 formed on the inner wall of the longitudinal tube portion 12.

Besides, the length of the parallel flanges 125 is designed such that, when the nebulizer 71 is inserted into the longitudinal tube portion 12 until it reaches and contacts the bottom ends of the parallel flanges 125, the valve plate 41 has been pushed to a desired height. The parallel flanges 125 not only guide sliding movement of the valve 4 but also limit an upward movement of the nebulizer 71.

The gasket 5 is a thin sheet disposed between the valve seat 126 and the valve plate 41 and is formed with a long narrow through hole 51 for extension of the valve extension 42. The gasket 5 is made of silica gel, rubber, or other elastomers. Since the gasket 5 is compressible, and since the spring 3 urges the valve plate 41 to move to the bottom opening 121, the compressible gasket 5 disposed between the valve plate 41 and the bottom opening 121 can seal the bottom opening 121 to prevent gas leakage between the transverse tube portion 11 and the longitudinal tube portion 12.Moreover, the gasket 5 can reduce frictional wear between the valve plate 41 and the valve seat 126 due to frequent opening and closing movements of the valve plate 41.

The Tee connector 100 further comprises a bottom cover 6 having a connection end 61 and a bottom plate end 62, as shown in Fig. 2. The connection end 61 can be connected to the Tee-shaped tube 1. In this embodiment, the Tee-shaped tube 1 has a mushroom-shaped protrusion 112 formed on an outer surface thereof. The connection end 61 can be sleeved on the protrusion 112. The bottom plate end 62 is for closing the bottom end 122 of the longitudinal tube portion 12 when the Tee connector 100 is not in use.

Because the spring 3, the valve 4 and the gasket 5 can be assembled inside the Tee-shaped tube 1 through the top opening 111, and the top opening 111 can be closed by the cover 2, the Tee connector 100 of the present invention can be assembled easier compared to the prior art.

Furthermore, as best shown in Fig. 5, since the valve extension 42 is hollowed to have an inverted U-shape and to form the two extension legs 420, the material cost can be reduced and resisting forces subjected by the gas flowing through the longitudinal tube portion 12 from the nebulizer 71 can be lowered.

## Claims

1. A Tee connector (100) for supplying aerosol, comprising:
a Tee-shaped tube (1) having a transverse tube portion (11) and a longitudinal tube portion (12), said transverse tube portion (11) having inlet and outlet ports and being formed with a top opening (111) and a bottom opening (121), said bottom opening (121) being aligned with said top opening (111) and being connected fluidly to said longitudinal tube portion (12);
a cover (2) closing detachably said top opening (111);
a valve (4) including a valve plate (41) which is movable downward to close said bottom opening (121) and upward to open said bottom opening (121); and
a spring (3) disposed within said transverse tube portion (11) and urging said valve plate (41) so that said valve plate (41) is moved downward to close said bottom opening (121).

2. The Tee connector (100) of Claim 1, wherein said valve (4) further includes a valve extension (42) extending from said valve plate (41) into said longitudinal tube portion (12).

3. The Tee connector (100) of Claim 2, wherein said valve extension (42) is guided slidably by said longitudinal tube portion (12).

4. The Tee connector (100) of Claim 3, wherein said valve extension (42) includes two extension legs (420).

5. The Tee connector (100) of Claim 4, wherein said longitudinal tube portion (12) has an inner wall formed with two guiding grooves (124) to guide said extension legs (420), respectively.

6. The Tee connector (100) of Claim 5, wherein said longitudinal tube portion (12) further has two pairs of parallel flanges (125) formed on said inner wall, each pair of said parallel flanges (125) confining one of said guiding grooves (124).

7. The Tee connector (100) of Claim 1, wherein said cover (2) includes a top plate (21), a pin portion (22) downwardly extending from said top plate (21) toward said valve (4), and a surrounding wall (23) surrounding said pin portion (22) and extending downwardly from said top plate (21), said pin portion (22) having a bottom end to contact and limit an upward movement of said valve plate (41) when said valve plate (41) moves upward.

8. The Tee connector (100) of Claim 7, wherein said spring (3) has top and bottom ends respectively abutting against said top plate (21) and said valve plate (41).

9. The Tee connector (100) of Claim 2, wherein said transverse tube portion (11) has a valve seat (126) disposed around said bottom opening (121) and protruding from an inner wall of said transverse tube portion (11), said valve plate (41) being seated on said valve seat (126) to close said bottom opening (121).

10. The Tee connector (100) of Claim 9, further comprising a gasket (5) disposed between said valve seat (126) and said valve plate (41).

11. The Tee connector (100) of Claim 10, wherein said gasket (5) is formed with a through hole (51) for extension of said valve extension (42).

12. The Tee connector (100) of Claim 1, wherein said spring (3) has a diameter larger than an inner diameter of said transverse tube portion (11).

13. The Tee connector (100) of Claim 1, wherein said valve plate (41) has a top face, and an angularly extending arc-shaped flange (43) protruding upwardly from said top face, said spring (3) being a coiled spring that has a bottom end disposed around and contacting said arc-shaped flange (43).
